# EUROPEAN PATENT SPECIFICATION

(11) **EP 3 577 537 B1**
(45) Date of publication and mention of the grant of the patent: **26.07.2023**
(21) Application number: 18748198.1
(22) Date of filing: 29.01.2018
(51) Int. Cl.: G06F 3/01, G06F 1/32, A61B 5/11, A61B 5/00, A61B 5/0205, A61B 5/024, G06F 1/3215, G16H 40/67, G06F 1/3206

(54) **DEVICE AND METHOD FOR MOTION DETECTION**
VORRICHTUNG UND VERFAHREN ZUR BEWEGUNGSERKENNUNG
DISPOSITIF ET PROCÉDÉ DE DÉTECTION DE MOUVEMENT

(30) Priority: 31.01.2017 SE 1750078
(43) Date of publication of application: 11.12.2019
(73) Proprietor: Outsmart AB, 211 19 Malmö (SE)
(72) Inventor: LEANDERSSON, Carl Fredrik, 246 57 Barsebäck (SE)
(74) Representative: Zacco Sweden AB
(86) International application number: PCT/SE2018/050062
(87) International publication number: WO 2018/143871

(56) References cited:
- WO-A2-2012/170924
- US-A1- 2010 304 754
- US-A1- 2013 091 503
- US-A1- 2015 100 245
- US-A1- 2015 277 540
- US-A1- 2016 018 872

## Description

### TECHNICAL FIELD

The invention pertains to the field of detection of motions. More particularly, it relates to device and methods for detecting motions of autonomous wearables.

### BACKGROUND

Activity trackers have become increasingly popular, and the features and functionality provided by such devices continue to expand to meet the needs and expectations of many consumers. To be able to detect and register a motion an activity tracker needs to be awake and ready to start registering a motion. Due to this feature some traditional activity trackers drain battery resources. One solution is to manually determine when to detect and register a motion, e.g. by pressing a button, a switch or something alike. However, this solution requires action of the user before a motion can be registered. Moreover, the user needs to remember to activate the device before an activity can be registered.

Hence, there is a need for an autonomous activity tracker that utilizes the power resources in a more efficient way.

One example of art in the field is US2015/277540 A1 disclosing power management for a wearable apparatus. A sensor may determine that a motion does not signify a gesture (e.g., flipping a wrist, a hand, etc.) or a user activity (e.g., walking, running, placing a hand on a desk, viewing a display, etc.), but rather is noise (e.g., a tremble, etc.) and refrains from forwarding a wake up signal to, for example, a processor of the apparatus to process the legitimate sensor data. In addition, the sensor may include a component to process the legitimate sensor data (e.g., to determine context), to specify a power mode in response to processing the legitimate sensor data, and/or to implement the power mode. Another example of art in the field is US2016/018872 A1 disclosing raise gesture detection in a device which can be executed on a low-power coprocessor (e.g., a motion coprocessor) of the device. Accordingly, when the device is not being actively used, a main or central processor of the device can be placed into a low-power, or sleep, state to further reduce power consumption of the device. Detection of a raise gesture by algorithms executing on the coprocessor can be used to wake the applications processor.

### SUMMARY

The scope of the invention is defined by the appended claims. The following description and drawings are present for illustration purposes only and shall aid the skilled reader in understanding the claimed invention, the scope of which is set out only in the appended claims.

In the present disclosure, a motion registration device, a method in a motion registration device and a piece of cloth with a motion detecting device are presented that provide for mechanisms that utilize resources of the battery in a more efficient way such that it does not need to be recharged as often.

This objective is obtained by a motion registration device as defined in appended claim 1.

It is provided for an autonomous tracking device that registers movements on its own initiative. Moreover, the motion detection can be designed to be waterproof. Furthermore, the device shuts down and saves battery when a movement pattern representing a movement that should not be registered is detected.

According to aspects, the plurality of movement patterns comprises a plurality of pre-defined movement patterns.

Hence, the accuracy of the determination of a movement pattern is increased.

According to further aspects, the control unit is further configured to determine the activity type and/or the false alarm event based on at least the determined movement pattern.

It is provided for that each movement pattern is associated with different categories of activity types and or false alarm events.

According to further aspects, the control unit comprises a data storage configured to store movement data associated with the plurality of movement patterns. Moreover, to determine a movement pattern further comprises to compare at least the received sensor data with movement data stored in the data storage.

No external device needs to be contacted in order to compare a sampled movement pattern with a plurality of movement patterns.

According to further aspects, the motion registration device further comprises a radio communication interface configured to communicate with at least a remote entity.

It is provided for wireless data communication to a remote entity.

According to further aspects, related to the aspects where the motion registration device comprises a communication interface, to determine a movement pattern may further be based on at least a signal received by the radio communication interface.

Hence, a variety of movement patterns can be determined.

According to the previous mentioned aspects, to determine a movement pattern may further comprise to transmit from the sensors received sensor data to at least a remote entity via the radio communication interface. It further comprises to receive from the at least a remote entity at least one of a movement pattern out of the plurality of movement patterns, an activity type of the motion registration device, and a false alarm event.

Flexibility is given as to where the determination of a movement pattern takes place. According to further aspects, the control unit is further configured to at least one of:
- control the one or more sensors to enter a sensor sleep mode when entering the motion registration device sleep mode.
- control the one or more sensors to exit the sensor sleep mode;
- enter a first control unit sleep mode when entering the motion registration device sleep mode;
- exit the first control unit sleep mode;
- exit the first control unit sleep mode in response to a trigger;
- exit the motion registration device sleep mode.

It is provided for different stages of power saving modes.

According to the previous aspects, the motion registration device further comprises a timer. The timer is configured to count down the sleep period and to provide the trigger to the control unit to exit the control unit sleep mode when the sleep period has elapsed.

Flexibility is given in how to set the duration of power saving modes.

According to further aspects, the control unit is further configured to exit a second control unit sleep mode in response to receiving the sensor data and/or notification that sensor data is available from the one or more sensors. The control unit is according to these aspects also configured to enter the second control unit sleep mode in response to exiting the motion registration device sleep mode.

Hence, different levels of power saving modes are available.

According to further aspects, each of the at least the first movement pattern is associated with a respective false alarm event, wherein the respective false alarm event is associated with a corresponding false alarm sleep period, and wherein the sleep period is the corresponding false alarm sleep period.

The power saving modes can be individualized and depend on different movement patterns.

According to further aspects, at least one of the first movement pattern is associated with a movement pattern of a running laundry machine.

Hence, it is provided for a motion registration device that autonomously put itself into a power saving mode when it is washed in a washing machine. Moreover, it autonomously exits the power saving mode when the washing procedure is finished and a movement pattern that is not a false alarm is detected.

According to further aspects, at least one of the one or more sensors further comprises a processor and a sensor data storage. The sensor data storage is configured to store basic movement patterns. The processor is configured to determine a basic activity type and a basic activity type estimate based on the stored basic movement patterns. The processor is further configured to transmit the determined basic activity type and the basic activity type estimate to the control unit.

According to the previous aspects, the control unit may further be configured to receive at least a basic activity type and/or at least a basic activity type estimate from at least one of the one or more sensors. The control unit may also be configured to determine the activity type and/or false alarm event based on at least the basic activity type and/or the basic activity type estimate.

According to further aspects, at least one of the one or more sensors is a Global Navigation Satellite System, GNSS, receiver.

According to the previous aspects, the sensor data may comprise received Global Navigation Satellite System, GNSS, signal strength.

Hence, a variety of off the shelf sensors and sensor systems can be integrated in the motion registration device.

According to further aspects, where the motion registration device comprise a radio communication interface configured to communicate with at least a remote entity, the motion registration device further comprise a data storage configured to store sensor data. Moreover, the control unit is configured to in response to determination of the second movement pattern save the sensor data associated with a movement of the motion registration device in the data storage. Furthermore, the control unit is further configured to transmit the saved sensor data to at least a remote entity via the radio communication interface.

A local memory provides for flexibility in time when it is suitable to transfer registered movement of the motion detection device to a remote entity.

According to further aspects, where the motion registration device comprises a radio communication interface configured to communicate with at least a remote entity, the at least a third movement pattern out of the plurality of movement patterns is associated with an undetermined movement pattern of the motion registration device. Moreover, the control unit is configured to in response to determination of the third movement pattern send at least the received sensor data via the radio communication interface to at least a remote entity. Moreover, the control unit is further configured to receive via the radio communication interface from the at least a remote entity a decision whether the movement pattern of the motion registration device is of at least a first movement pattern or a second movement pattern.

Hence, further flexibility is given as to where the determination of a movement pattern takes place and how this process is performed. Utilizing this solution it is provided for a connection with updated data bases containing a huge amount of movement patterns. It is also possible to have a solution where movement patterns specifically associated with the user is set / administrated via a remote entity.

According to further aspects, where the motion registration device comprises a radio communication interface configured to communicate with at least a remote entity, the control unit is further configured to receive via the radio communication interface from the at least a remote entity at least one movement pattern. The control unit is also configured to store the received at least one movement pattern in the data storage.

Hence, it is provided for functions such that the motion detecting device can be updated. Other aspects are related to a piece of cloth with a motion detecting device, as described above, attached to it.

Thereby, relevant movements of a user are directly registered by the autonomous motion detecting device when the piece of cloth is worn. Moreover, events that are not of interest for the user to register, i.e. false alarm events, are not registered and battery saving modes is entered by the device.

The present disclosure also relates to a method in a motion registration device as defined in appended claim 9.

The same advantages and benefits are obtained as for the corresponding features of the previously discussed motion registration device.

According to further aspects, the plurality of movement patterns comprises a plurality of pre-defined movement patterns.

According to further aspects, the method further comprises determining the activity type and/or the false alarm event based on at least the determined movement pattern.

According to further aspects, the determining movement pattern of the motion registration device further comprises comparing at least the obtained sensor data with movement data associated with the plurality of movement patterns.

According to further aspects, the determining movement pattern of the motion registration device is further based on at least a received signal.

According to further aspects, the determining movement pattern of the motion registration device further comprises transmitting the obtained sensor data to at least a remote entity. The determining movement pattern also comprises receiving from the at least a remote entity at least one of a movement pattern out of the plurality of movement patterns, an activity type of the motion registration device and a false alarm event.

According to further aspects, the method further comprises at least one of:
- controlling the one or more sensors to enter a sensor sleep mode when entering the motion registration device sleep mode;
- entering a first control unit sleep mode when entering the motion registration device sleep mode;
- exiting the motion registration device sleep mode;
- controlling the one or more sensors to exit the sensor sleep mode when exiting the motion registration device sleep mode;
- exiting the first control unit sleep mode when exiting the motion registration device sleep mode; and
- exiting the first control unit sleep mode in response to a trigger.

According to the previous aspects, the method further comprises counting down the sleep period and providing the trigger to the control unit to exit the control unit sleep mode when the sleep period has elapsed. Both the counting down and the providing the trigger are performed by a timer.

According to further aspects, the method further comprises exiting a second control unit sleep mode in response to the obtaining of sensor data and/or notification that sensor data is available from the one or more sensors. The method further comprises entering the second control unit sleep mode in response to exiting the motion registration device sleep mode.

According to further aspects, each of the at least the first movement pattern is associated with a respective false alarm event, wherein the respective false alarm event is associated with a corresponding false alarm sleep period, and the sleep period is the corresponding false alarm sleep period.

According to further aspects, at least one of the first movement pattern is associated with a movement pattern of a running laundry machine.

According to further aspects, the method further comprises providing at least a basic activity type and/or at least a basic activity type estimate from at least one of the one or more sensors. Moreover, the method further comprises determining the activity type and/or false alarm event based on at least the basic activity type and/or the basic activity type estimate.

According to further aspects, at least one of the one or more sensors is a Global Navigation Satellite System, GNSS, receiver.

According to the previous aspects, the sensor data may comprise received Global Navigation Satellite System, GNSS, signal strength.

According to further aspects, the method further comprises in response to determination of the second movement pattern registering sensor data associated with a movement of the motion registration device.

According to the previous aspects, the method may further comprise transmitting the registered sensor data to at least a remote entity.

According to further aspects, at least a third movement pattern out of the plurality of movement patterns is associated with an undetermined movement pattern of the motion registration device. Moreover, in response to determination of the third movement pattern, the method further comprises sending at least the obtained sensor data to at least a remote entity. It further comprises receiving from the at least a remote entity a decision whether the movement of the motion registration device is of at least a first movement pattern or a second movement pattern.

According to further aspects, the method further comprises receiving from the at least a remote entity at least one movement pattern and storing the received at least one movement pattern.

According to some aspects, the present disclosure also relates to computer readable storage medium, having stored there on a computer program which, when executed in a motion registration device, causes the motion registration device to execute the previously described methods.

The computer readable storage medium display advantages corresponding to the advantages already described in relation to the respective methods disclosed above.

### BRIEF DESCRIPTION OF THE DRAWINGS

The foregoing will be apparent from the following more particular description of the example embodiments, as illustrated in the accompanying drawings in which like reference characters refer to the same parts throughout the different views. The drawings are not necessarily to scale, emphasis instead being placed upon illustrating the example embodiments.
Figure 1 is an example scenario wherein a motion detection device communicates with remote entities;
Figure 2 is a further example scenario wherein an embodiment of the present teaching is attached to a garment and washed in a washing machine;
Figure 3 is a schematic drawing of a motion detection device, according to some of the example embodiments;
Figure 4 illustrates sensor and control unit activity as a function of time according to some of the examples disclosed;
Figure 5 is a schematic drawing of an activity sensor, according to some of the example embodiments;

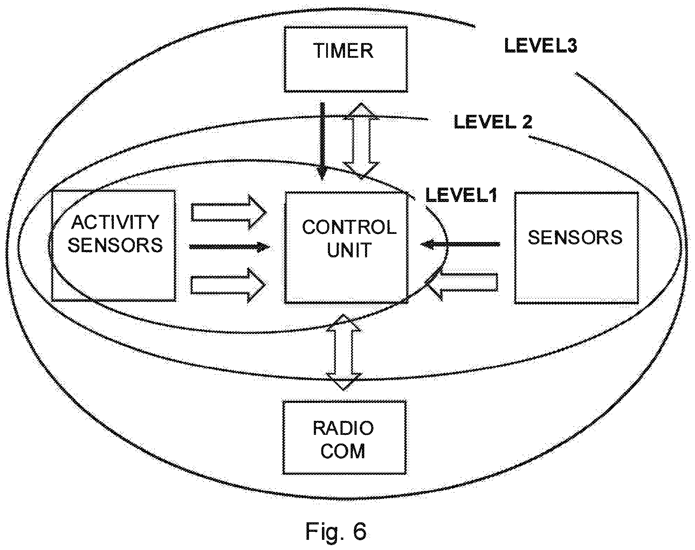

Figure 6 is a schematic signaling scheme according to the present teachings;
Figure 7a-c is other schematic signaling schemes according to the present teachings;
Figure 8 is a flowchart illustrating method steps according to some of the example embodiments;
Figure 9 is a flowchart showing further aspects of methods according to the present teachings;
Figure 10 is a flowchart showing further aspects of methods according to the present teachings;
Figure 11 is a flowchart showing further aspects of methods according to the present teachings;
Figure 12 is a flowchart showing further aspects of methods according to the present teachings;
Figure 13 is a flowchart showing further aspects of methods according to the present teachings; and
Figure 14 is a flowchart showing further aspects of methods according to the present teachings.

### DETAILED DESCRIPTION

Aspects of the present disclosure will be described more fully hereinafter with reference to the accompanying drawings. The apparatus and method disclosed herein can, however, be realized in many different forms and should not be construed as being limited to the aspects set forth herein. Like numbers in the drawings refer to like elements throughout.

The terminology used herein is for the purpose of describing particular aspects of the disclosure only, and is not intended to limit the invention. As used herein, the singular forms "a", "an" and "the" are intended to include the plural forms as well, unless the context clearly indicates otherwise.

In some of the figures, operations and modules are illustrated with a solid border or with a dashed border. The operations and modules which are illustrated with solid border are operations which are comprised in the broadest example embodiment. The operations and modules which are illustrated with dashed border are example embodiments which may be comprised in, or a part of, or are further embodiments which may be taken in addition to the operations and modules of the broader example embodiments. It should be appreciated that the operations do not need to be performed in the precise order that is presented herein.

Operations may be performed simultaneously or in different orders within the scope of protection. Furthermore, it should be appreciated that not all of the operations need to be performed. Hence, the example operations may be performed in any order and in any combination apparent to the person skilled in the art. Moreover, all figures are illustrative which means that further devices and method steps can be incorporated without exceeding the scope of protection.

Some of the example embodiments presented herein are directed towards autonomous activity trackers that utilize its power resources in an efficient way. As part of the development of the example embodiments presented herein, a problem will first be identified and discussed.

The presented devices and methods disclose how to utilize the power resources in a more efficient way. This is performed by labeling specific movement patterns, e.g. running, swimming, washing machine circulation, etc. to be of a specific activity type or of a false alarm event. The false alarm events are typically such activities that the user is not interested in registering. The inventor has realized that when a movement pattern of the user corresponds to a false alarm event then the motion registration device can enter a sleep mode for a predetermined time and thereby the battery is not drained as quickly.

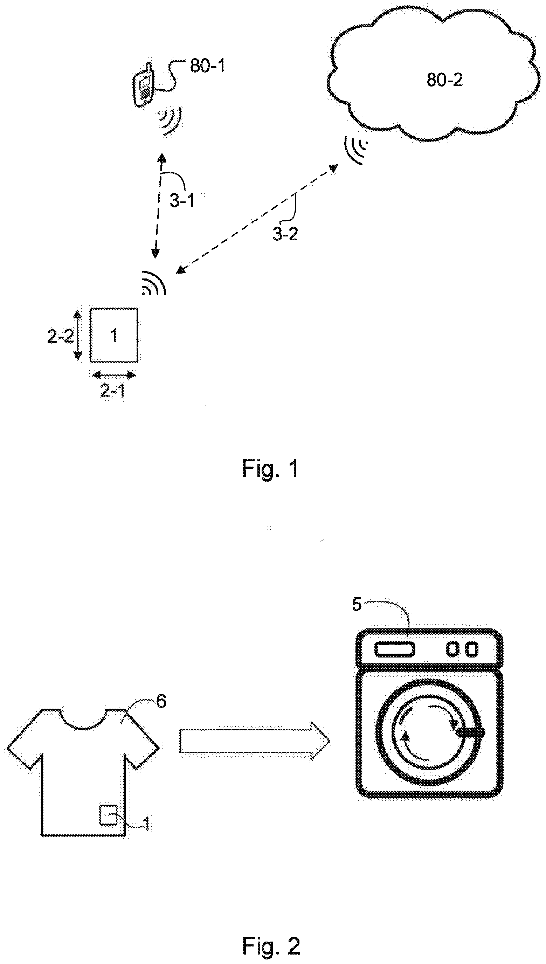

To facilitate the understanding of the proposed motion registration device different aspects relating to activity trackers is further elaborated in relation to a typical scenario as illustrated in Figure 1.

Figure 1 shows a motion registration device 1 and two remote entities 80-1, 80-2. The motion registration device may be a portable electronic device, a wearable electronic device, or other type of device. By way of example and not by way of limitation, the motion registration device may be an electronic device, a mechanical device, or an electromechanical device. The motion registration device can be a separate entity or it can be integrated in another device. Non-limited examples of where the motion registration device may be incorporated are mobile phones, personal digital assistants, music players, timekeeping devices, health monitoring devices, tablet computers, laptop computers, glasses (electronic or otherwise), portable storage devices, and the like.

The remote entities 80-1, 80-2 can be any electronic device configured to receive and transmit 3-1, 3-2 communication from/to the radio communication interface of the motion registration device. They can for example be a cloud storage mediums, a central server, mobile terminal, mobile phone, smart watch, tablet, usb-stick, laptop, a stationary or mobile wireless device for machine-to-machine communication, personal digital assistants, music players, timekeeping devices, health monitoring devices, different computers, glasses (electronic or otherwise), portable storage devices an integrated or embedded wireless card, an externally plugged in wireless card, a dongle, external servers etc.

The basic concept of a tracking device 1 is to register data associated with movement of the device 2-1, 2-1 and then transfer 3-1, 3-2 it to a remote entity where the user of the tracking device has access.

In this typical scenario the user is a keen runner and he is very interested of logging this activity. He sometimes goes for a walk but this is of no interest to him to keep track of. The motion detection device is small, robust and waterproof and can be an integrated part of the user's sporting garment. The user does not have to worry of starting the device or switching it off. The device is autonomous and starts to register the movement pattern of running as soon as its starts. If the device recognize the movement pattern of walking it autonomously goes into a sleep mode for a pre-set period of time in order to save power. After the workout the user returns to home and the garment is thrown in a corner. However, being at rest and having access to a reliable and fast wireless internet connection the motion detection device autonomously starts to transmit the records of the workout to a central server where the user has access. After a while the garment is to be washed. The motion detection device recognizes the motion pattern of a circulating washing machine and again goes into a sleep mode for a pre-set period of time in order not to drain the battery.

Available products can detect basic activities e.g. walking, running, biking, and swimming and label it as such or as an unknown activity. In the sequel, this will be denoted 1^{st} layer detection. In the proposed solution an off the shelf product can be used to wake up the main system (processor) via e.g. an interrupt signal.

In order not to drain the battery in vain, there is a need to quickly discriminate the movement from being a false alarm event. These false alarms might for example be associated with any logistic movement of the device or clothes where the device is fitted, washing machine (or dry tumbling) circulation, a short running distance, warm-up activities (before the actual activity begins), a car ride, flight transportation or boat transportation.

The inventor has realized that once the main system of the motion registration device is active, the proposed solution can be applied to refine the classification of the activity and shut-down the activity of the processor and sensors when a false alarm is detected. This discrimination is referred to as 2^{nd} layer detection.

There is also another outcome where the movement pattern is unknown. The motion registration device may then call a central server function (a remote entity), via an Internet connection (e.g. established through cellular communication or short-range air interface as, for example, Bluetooth or Wi-Fi or equivalent communication method). This is referred to as 3^{rd} layer detection.

If a valid movement is detected, the main system (processor) stays active and starts to log parameters related to the movement, as e.g. activity time, position, speed, accumulated distance.

If a false alarm is detected, the main system (the processor) and the sensor system (1^{st} layer detection) are set to deep sleep mode for a certain time, depending on the type of false alarm.

One example of a false alarm is given in Figure 2 where the motion registration device 1 is attached to a garment 6 and positioned in a washing machine 5. When the washing machine movement pattern is detected, a reasonable timer interval would be e.g. 30 minutes. When the time expires, the main system wakes up and enables the power to the sensor system. After enabling the sensor subsystem, the main system goes to sleep again and only wakes up if there is a new interrupt received from the sensor subsystem.

Hence, it is provided for a robust, simple and cheap autonomous motion detection device that with ease can be integrated in e.g. garments. The user does not need to pay any attention to it for a long time since it has low power consumption. Moreover, the device autonomously detects and registers by the user preferred movement patterns and sends the corresponding data to a remote entity where the user gets access to the data. Moreover, the device 1 can be waterproof and can be e.g. used during swimming or attached to garment 6 when being washed.

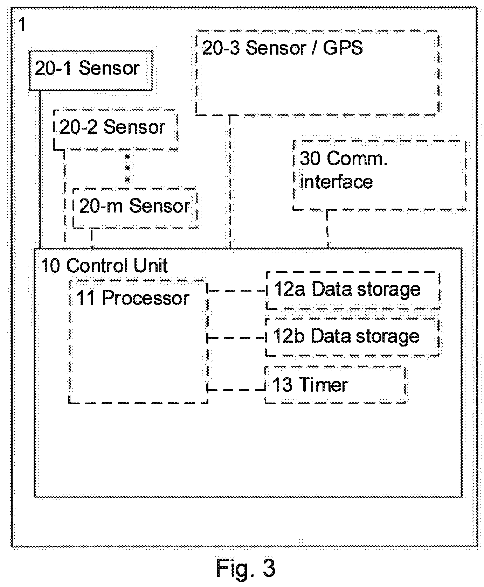

The proposed method will now be described in more detail referring to Figure 3.

Figure 3 shows a motion registration device 1, said device comprising one or more sensors 20-1, 20-2, ..., 20-m and a control unit 10. The motion registration device 1 may also comprise a radio communication interface 30 configured to communicate with at least a remote entity 80-1, 80-2. According to some aspects the control unit further comprises data storage/s 12a, 12b. The control unit may also comprise a timer 13.

The dashed boxes in the figure indicate that the features are optional and can be combined in various ways known to a person skilled in the art. Moreover, the data storages 12a, 12b and the timer 13 are comprised in the motion registration device, hence, in some embodiments some of these devices might be positioned outside the control unit 10.

According to aspects the communication interface comprises communication circuitry configured to receive and transmit signals either wirelessly, by wire, or both.

According to some aspects the control unit comprises a processor 11, this may be e.g. a Central Processing Unit, CPU, a Communication Central Processing Unit, CCPU, or an Application Central Processing Unit, ACPU. That is, the processing circuitry 12 may be any suitable type of computation unit, e.g. a microprocessor, digital signal processor, DSP, field programmable gate array, FPGA, or application specific integrated circuit, ASIC, or any other form of circuitry. It should be appreciated that the processing circuitry need not be provided as a single unit but may be provided as any number of units or circuitry.

The one or more sensors 20-1, 20-2, ..., 20-m is configured to provide sensor data associated with a movement of the motion registration device 1. In one exemplary at least one of the one or more sensors 20-3 is a Global Navigation Satellite System, GNSS, receiver, using for instance, the Global Positioning System, GPS, GLONASS, Quasi-Zenith Satellite System, QZSS, Galileo, Beidou systems or combinations of those. By utilizing a GNSS e.g. the distance and the velocity of the device can be registered.

Further examples of usable sensors are accelerometer, gyrocompass, pressure sensor, pulse detector, magnetometer, cellular modem to detect and classify the activity of the user and register related parameters or similar devices known to the person skilled in the art. The sensors are capable of detecting a sensor value and transmitting it to the control unit. The sensor/s can further be controlled by the control unit. For example, the control unit might be able to put the sensors in a sleep mode and to wake them.

The sensors do not consider personal statistics or configurations of a particular user, nor any kind of other learning that can be gained during the usage of the device, by a particular user. Hence, a variety of non-expensive off the shelf sensors and sensor systems can be integrated in the motion registration device as a first level estimator. Due to the low price a consumer can easily afford several devices that can be attached to e.g. different garments.

Sensor data may be motion data of a movement pattern or gesture pattern sufficient to characterize the corresponding activity type. In one exemplary the sensor data is raw sensor data e.g. amplitude and/or time samples. Other parameters of interest to identify the movement pattern may be arm-frequency, arm above water and swimming-style for swimming. Parameters related to running activities are e.g. pace, distance and step-length. In another exemplary the sensor data is codified as a model and parameters, containing the motion state machine and parameters defining the activity type and the registered performance of the user.

Since various sensors can be employed multiple alternative input data/signals can be utilized to determine the movement pattern which vouch for an accurate prediction.

The control unit 10 in Figure 3 is configured to receive the sensor data from the one or more sensors 20-1, 20-2, ...., 20-m. Hence, the registered parameter values are transmitted to the control unit. In one exemplary the values are received by an ACPU.

The control unit 10 is further configured to determine a movement pattern of the motion registration device 1 out of a plurality of movement patterns of the motion registration device 1 based on at least the received sensor data. A movement pattern is a specific motion that can be associated with a specific activity or gesture. For example one movement pattern represent walking whereas a second represents bicycling and a third might represent circulation in a washing machine. In other words the control unit performs activity and gesture recognition by utilizing the received sensor data to determine the movement pattern of the device. This is referred to as 2^{nd} layer detection.

According to some aspects, the plurality of movement patterns comprises a plurality of pre-defined movement patterns.

Pre-defined movement patterns may be movement patterns that are defined as default when the device is delivered. The pre-defined movement patterns can also refer to movement patterns specifically chosen by the user, e.g. the user is a darts enthusiast and wants to log this activity. Having a plurality of pre-defined movement patterns increases accuracy of the determination of a movement patterns since it is more likely that the correct movement pattern can be found. Moreover, with a vast range of movement patterns special gestures and movements may be identified.

In the case where the motion detection device is suitable for exercise registration the pre-defined movement patterns might correspond to the activities of walking, running, biking, or swimming (including sim-swimming style). Hence, at least a second movement pattern out of the plurality of movement patterns is associated with an activity type of the motion registration device. In other words, a second activity type is associated with an activity to be registered. In one exemplary the pre-defined activity types are generic. In another exemplary some or all of the pre-defined activities can be specific for the user, e.g. a user can decide that only swimming and running are of interest to log. According to further aspects, the ACPU can be used to combine the determined movement pattern with data in a personal activity profile and personal statistics stored in memory (personal library) as well as raw-data from the sensors. The quality of the personal data and motion library is iteratively improved as the total device is deployed to record the data and behavior of a particular user.

Hence, a variety of movement patterns can be determined.

In order to save power consumption, the device is capable of recognising activities that are not of interest for the user to log. This discrimination is performed in the 2nd layer detection. Thus, at least a first movement pattern out of the plurality of movement patterns is associated with a false alarm event and at least a second movement pattern out of the plurality of movement patterns is associated with an activity type of the motion registration device. In other words, the first activity type is associated with an activity type not to be registered and the second movement pattern is associated with an activity of interest for the user to log, e.g. running.

The false alarm events can for example correspond to washing machine (or dry tumbling) circulation or any logistic movement of the device or clothes where the device is fitted. It may further correspond to a short running distance or warm-up activities before the actual activity begins. According to aspects, the user can define those activities which are to be classified as false alarm events. For example, the user only wants to register his swimming sessions and not his fast running to the bus every morning. Hence, the user defines the movement pattern of swimming as a second movement pattern associated with the activity swimming and he defines the movement pattern of short, fast running as a first movement pattern associated with a false alarm.

Even further false alarm events can be e.g. car rides, flights and boat transportation.

According to some aspects, the activity type and/or the false alarm event is determined based on at least the determined movement pattern. Hence, each movement pattern is associated with different categories of activity types and/or false alarm events.

In order not to drain the battery in vain, there is a need to quickly discriminate the activity types to be logged from false alarm events. According to aspects in the presented subject matter the detection of a false alarm event is used to quickly discriminate e.g. washing machine motion and shut down the sensors and the ACPU for a certain sleep period. Hence, the control unit 10 configured to enter a motion registration device sleep mode for a sleep period in response to determination of the at least first movement pattern. A sleep mode is a mode wherein the device consumes considerably less power compared to when it is up and running. A sleep period is a period of time wherein the device is in sleep mode or a low-power consumption mode.

It is provided for a device comprising means suitable for sequentially assessing and improving activity recognition decisions, originating from a monolithic external device.

In other words, the disclosed motion detection device is an autonomous tracking device that registers movements on its own initiative. Hence, it is always ready without any external activation from e.g. a user or other remote entity. Furthermore, the device shuts down and saves battery when a movement pattern representing a movement that should not be registered, i.e. a false alarm event, is detected.

Since the device is autonomous it does not need to comprise any contacts, buttons (however it is not excluded). This makes it possible to manufacture devices that are non-expensive, robust, and water proof. These features make the device suitable for use while swimming, canoeing etc. Moreover, it stands being washed in a laundry machine.

The decision of which movement pattern that corresponds to the collected sensor data can be performed in various ways. A few examples are given in the following paragraphs however they should not be seen as limited.

According to some aspects the data storage 12a is configured to store movement data associated with the plurality of movement patterns. In other words, parameter values associated with a specific movement pattern is stored in the data storage and labelled as a movement pattern (e.g. circulation of a washing machine) and a activity type or false alarm event (i.e. false alarm event when the movement pattern is circulation of a washing machine). Other examples of movement data is pulse, arm-frequency, arm above water, pace, distance and step-length.

According to some aspects, the movement data is saved sensor data of known movement patterns.

A movement pattern can be associated with several different parameters in order to define the activity as precise as possible. As previously mentioned, the stored movement patterns can be generic to all users (e.g. the circulation of a washing machine). However, the stored movement patterns or parts thereof may also be personally configured to suit the user's specific desires in respect of which activities that should be logged.

The data storage 12a may be configured to store received or transmitted data and/or executable program instructions. The data storage 12a may be any suitable type of computer readable memory and may be of volatile and/or resident type. The data storage is accessible by the control unit and according to some aspects the processor.

According to some aspects, to determine a movement pattern further comprises to compare at least the received sensor data with movement data stored in the data storage 12a. The comparing can be performed by different methods, e.g. least square method/meaning, maximum likelihood, sequence estimation or any other suitable method know to the skilled person.

Hence, no external device needs to be contacted in order to compare a sampled movement pattern with a plurality of movement patterns to determine if the movement is an activity that is of interest to the user or if it is a false alarm event.

In one exemplary a Global Navigation Satellite System, GNSS, uses GPS which provides the sensor data as described above and by utilizing a GPS signal e.g. the velocity of the motion detection device can be detected which can be used in the determination of a correct movement pattern.

In a further exemplary, the sensor data comprises received GNSS signal strength which can provide information of the movement pattern. For example, the RSSI, Received Signal Strength Indication, may be evaluated. This can be relevant when looking at different swimming patterns since the RSSI is noticeably reduced (e.g. by 10-15 dB) when the device is positioned under water. When swimming is to be detected it can be relevant to wear several devices. One attached to the arm which is periodically located under water and one device attached to the swim wear which is constantly located under water.

Any kind of signal where e.g. the received signal strength, RSS, and reflected power can be determined can be used to support the classification of movement pattern. Hence, in an exemplary where motion registration device comprises a radio communication interface 30 which is configured to communicate with at least a remote entity 80-1, 80-2, to determine a movement pattern may further be based on at least a signal received by the radio communication interface 30. According to aspects, parameters from a cellular modem, can be used to support this classification, e.g., due to attenuation in water. The cellular modem deploys 2G/3G/4G/5G, NB-IOT or any other suitable communication technique known in the art. Similarly, parameters from other kinds of sensors, as microphone, proximity, temperature, pulse, or other, can also be used to support the classification.

According to some aspects it is desirable to let another remote entity do the determination. In other words, the motion registration device comprises a radio communication interface 30 which is configured to communicate with at least a remote entity 80-1, 80-2. Further, to determine a movement pattern further comprises to transmit from the sensors 20-1, 20-2, ..., 20-m received sensor data to at least a remote entity 80-1, 80-2 via the radio communication interface 30. That is the control unit transmits data provided by the sensors via the radio communication interface 30. Processing of the sensor data is performed in the remote entity. The motion detection device is provided with either a movement pattern that can be matched to one of the movement patterns stored in the motion detection device and/or it is provided an activity type/false alarm event. In other words, in order to determine the movement pattern the motion detection device receives from the at least a remote entity 80-1, 80-2 at least one of a movement pattern out of the plurality of movement patterns, an activity type of the motion registration device and a false alarm event. Thus, flexibility is given as to where the determination of a movement pattern takes place.

According to even further aspects, if the activity type determined by the remote entity is not comprised in the plurality of movement patterns stored in the data storage 12a then it is saved in the data storage 12a.

It should be appreciated that the radio communication interface 30 may be comprised as any number of transceiving, receiving, and/or transmitting units or circuitry. It should further be appreciated that the radio communication interface 30 may be in the form of any input/output communications port known in the art. The radio communication interface 30 may comprise RF circuitry and baseband processing circuitry. The communication interface 30 may support either wireless and/or wired communication. Examples of wireless communication may be Global System for Mobile Communication, GSM, Bluetooth, or Narrowband Internet of Things, NB-loT.

In one exemplary the movement pattern cannot be determined accurately. This might for example be in a situation where the detected sensor data does not match any of the pre-defined movement patterns. In this case the movement pattern is said to be undetermined. In other words, at least a third movement pattern out of the plurality of movement patterns is associated with an undetermined movement pattern of the motion registration device 1. That is, a third activity type of the motion registration device is associated with an activity type that is to be determined by at least a remote entity 80-1, 80-2. Hence, when e.g. an external server has to be contacted in order to determine the movement pattern or activity type it is referred to as 3rd layer detection.

Moreover, the control unit 10 is configured to, in response to determination of the third movement pattern; send at least the received sensor data via the radio communication interface 30 to at least a remote entity 80-1, 80-2. Hence, the detected parameter values are transmitted to a remote entity for an external evaluation. The control unit is further configured to receive via the radio communication interface 30 from the at least a remote entity 80-1, 80-2 a decision whether the movement pattern of the motion registration device is of at least a first movement pattern or a second movement pattern. Hence, according to some aspects and in the event that the second layer detection fails to classify the activity with good certainty, the third layer is invoked and raw sensor data is sent via Internet connectivity peripheral to a cloud computing server. The result is then feed back to the device (ACPU) via the same connectivity interface.

Phrased differently and according to further aspects the ACPU cannot decide on the activity and it will therefor call a central server function, via an Internet connection. This connection can be established through e.g. cellular communication or short-range air interface as, for example, Bluetooth or Wi-Fi, or other connections known in the art.

When the motion detection device then received a decision whether the movement pattern of the motion registration device is of at least a first movement pattern or a second movement pattern it can then proceed with the process of either setting the device in sleep mode in response to a first movement pattern, e.g. a false alarm event, or to start logging the movement of the device in response to a second movement pattern corresponding to an activity type.

Hence, further flexibility is given as to where the determination of a movement pattern takes place and how this process is performed. By utilizing this solution it is provided for a connection with updated data bases containing a huge amount of movement patterns. It is also possible to have a solution where movement patterns specifically associated with the user is set / administrated via a remote entity.

In order to keep the data storage (12a) comprising the plurality of movement patterns up to date the motion detection device may be able to update itself. In other words, according to aspects, the control unit (10) is further configured to receive via the radio communication interface (30) from the at least a remote entity (80-1, 80-2) at least one movement pattern. The control unit may further be configured to store the received at least one movement pattern in the data storage (12a).

Hence, the movement patterns may be downloaded or uploaded to a remote entity from the motion detection device. Moreover, in the exemplary where an undetermined movement pattern has been encountered and a remote entity has been able to identify the pattern, it may be desirable to store the movement pattern locally. In this way, the next time the same movement pattern is encountered, power is saved since a remote entity does not need to be contacted.

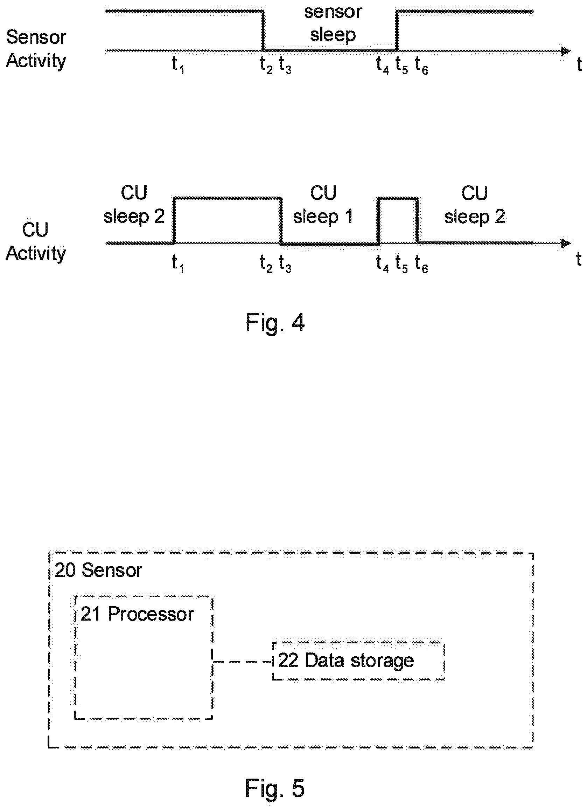

In order to limit the power consumption, the autonomous motion detection device shuts down whenever possible. Different sleeping modes will be exemplified in relation to Figure 4, where the sensor and control unit activity is shown as a function of time, t. It is appreciated that the embodiment of Figure 4 and the corresponding text should not be seen as limiting. That is, the order in which the different devices are put to sleep and are awakened can be altered in any way known to the skilled person. The actions may also be performed simultaneously. Moreover, not all functions described needs to be present at the same time and further functions can be added within the scope of protection.

Starting to the left in Figure 4, the motion detection device is not moving, the sensors are awake in order to anticipate a motion. The control unit 10, CU is in a second control unit sleep mode to save power. According to one aspect the second control unit sleep mode is a deep sleep mode where the control unit stays until it is triggered by an input from the sensors.

At the time t1 the one or more sensors detects a movement of the motion detection device and notifies the control unit. The control unit may for example be woken by an interrupt signal from the sensor device. This signal itself does not carry sensor data. The interrupt signal can be part of a bus or separate from the bus between the sensor and control unit. The interrupt service routine, SW, running on the control unit may then ask the sensor (on the bus) for the sensor data. Such implementation provides for power efficiency. That is, the control unit is configured to exit a second control unit sleep mode in response to receiving the sensor data and/or notification that sensor data is available from the one or more sensors.

The sensor data is analyzed and the control unit determines a movement pattern. In this exemplary it is assumed that the movement pattern is a first movement pattern, i.e. it corresponds to a false alarm event (e.g. washing machine circulation). The motion registration device enters a sleep mode to save power. Hence, at t2 the control unit 10 is configured to control the one or more sensors 20-1, 20-2, ..., 20-m to enter a sensor sleep mode when entering the motion registration device sleep mode. According to aspects, the sensors are off in the sensor sleep mode and do not register any motion. The control unit is further configured to enter a first control unit sleep mode when entering the motion registration device sleep mode at t3. According to aspects, the control unit is turned off in this sleep mode. Further, a timer 13 is configured to count down the sleep period, which in this exemplary last between t3 and t4. The timer may be a Real Time Clock, RTC. To further reduce power consumption the timer may also be a low-power timer countdown. At t4 the timer is configured to provide the trigger to the control unit 10 to exit the control unit sleep mode when the sleep period has elapsed. Hence, the control unit is configured to exit the motion registration device sleep mode. In the exemplary in Figure 4, this means exit the first control unit sleep mode or exit the first control unit sleep mode in response to a trigger at t4 and control the one or more sensors 20-1, 20-2, ..., 20-m to exit the sensor sleep mode at t5. Hence, the sensors are up and running and ready to detect movements of the motion detection device. At t6 the control unit is configured to enter the second control unit sleep mode in response to exiting the motion registration device sleep mode. Hence, the control unit returns to deep sleep. According to different aspects it stays in deep sleep until it gets triggered by the sensors. It may also be awakened by some other event, e.g. communication with another (remote or a nearby) entity or manual interaction from the user, e.g., charging.

Hence, it is provided for an autonomous motion detection device that by itself goes into different stages of power saving modes when different parts of the device does not need power supply.

To further increase the flexibility, the sleep period, can according to aspects be individualized and depend on different false alarm events that are detected. In other words, each of the at least the first movement pattern is associated with a respective false alarm event, wherein the respective false alarm event is associated with a corresponding false alarm sleep period, and wherein the sleep period is the corresponding false alarm sleep period. For example, if it is detected that the motion detection device is in the laundry machine a sleep period of 40 minutes might be appropriate, if instead a warm up activity is registered a sleep period of 5 minutes might be expected.

Thus, flexibility is given in how to set the duration of power saving modes.

The exemplary described in relation to Figure 4 shows a scenario where a false alarm event is determined. The different sleep modes can also be utilized when a second movement pattern is detected. For example, assume the determined movement pattern corresponds to running. According to some aspects, all sensors except the Global Navigation Satellite System, GNSS, system can be turned off. According to further aspects, the user can be an advanced runner and has a profile (in the personal library) where the number of steps should be registered as well; hence the sensor registering the number of steps should not be controlled to enter a sleep mode by the control unit. Another example is swimming where the relevant sensors to keep awake might be the ones that can detect the number of arm strokes. Hence, according to aspects each respective second movement pattern is associated with a data capturing and recording profile stating which sensors those are relevant to keep awake.

These features provides for even further reduction of the power consumption. A local memory provides for flexibility in time when it is suitable to transfer registered movement of the motion detection device to a remote entity.

According to further aspects, the off the shelf sensors 20 integrated in the motion detection device may be a sensor-HUB 20 also referred to as activity sensor 20. This is illustrated in Figure 5 where at least one of the one or more sensors 20 further comprise a processor 21 and a sensor data storage 22, wherein the sensor data storage 22 is configured to store basic movement patterns. Basic movement patterns refer to standard movement patterns such as e.g. running, bicycling, walking. That is, a basic movement pattern is a movement pattern that is fairly easy to recognize and/or a movement pattern of the device that is very likely to occur. If none of these movement patterns are recognised the movement pattern may be defined as undefined movement. No personal settings are available at this level. The processor 21 is configured to determine a basic activity type and a basic activity type estimate based on the stored basic movement patterns. Hence, the sensor has its own basic activity recognition process. The basic activity type estimate gives a probability of how likely it is that the determined movement pattern is correct. Thus, it is an uncertainty factor, e.g. a maximum likelihood ratio.

The processor 21 and sensor data storage 22 may be of the same types as described in reference to the processor and data storages of the control unit device.

The processor 21 of the sensor if further configured to transmit the determined basic activity type and the basic activity type estimate to the control unit 10. In other words, the sensors provide the control unit with activity type and probability that the activity type is correct chosen and.

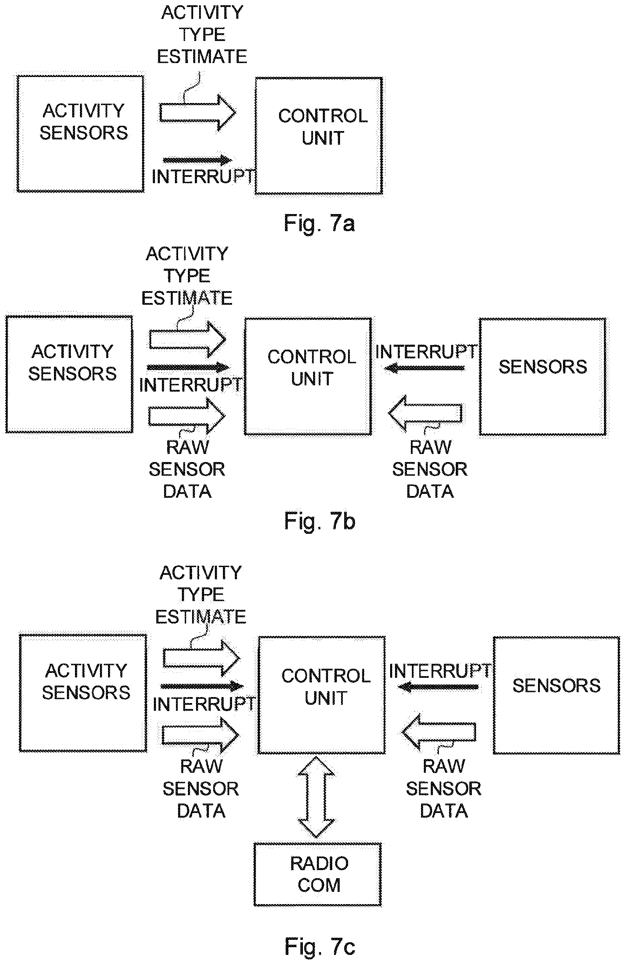

Utilizing a sensor-HUB which is able to provide the control unit with at least a basic activity type is referred to the 1st layer detection and also figuratively illustrated in Figure 7a.

Further, and as illustrated in Figure 7b, the control unit 10 is configured to receive at least a basic activity type and/or at least a basic activity type estimate from at least one of the one or more sensors. The control unit is further configured to determine the activity type and/or false alarm event based on at least the basic activity type and/or the basic activity type estimate. If the activity type estimate indicates that the correct activity type has been chosen the control unit does not need to do its own determination, hence power is saved. Instead the control unit starts logging the activity directly. If the activity type estimate indicates a certain uncertainty the control unit may control if the activity type is found in a personal library/data storage configured with common activity types of the user. If the activity type is present in the personal library then it is likely that the correct activity type has been established and the control unit may decide to start logging instead of doing a full determination based on the sensor data. Hence, power can be saved. In other cases where e.g. the basic activity type is undetermined or where the activity type estimate shows a low likelihood of correctness then further sensors may be started, the raw unprocessed/raw sensor data from the activity sensor is provided and the control unit determines the movement pattern as described above. This is referred to 2nd layer detection, cf. Figure 7b.

If uncertainty still exists, the 3rd layer detection is invoked by sending the movement's characteristics, e.g. model parameters to a remote entity e.g. the central server via internet, for a judgment, as described above. This is figuratively illustrated in Figure 7c.

Hence, it is provided for a motion registration device utilizing the potential of off the shelf products. Moreover, in the situations where the off the shelf sensors fail to determine a correct movement pattern the disclosed product refines the determination of a correct movement pattern in low power consumption manner.

When a second movement pattern is determined the relevant sensors are triggered to start logging as described above. For example, if a second movement pattern corresponding to running is determined, the Global Navigation Satellite System, GNSS (using e.g. GPS) starts to log the user's position. In other words and according to further aspects, the motion registration device 1 further comprises a data storage (12b) configured to store sensor data. This data storage can be separate from the data storage 12a or it could be the same. Further, the control unit 10 is configured to in response to determination of the second movement pattern save the sensor data associated with a movement of the motion registration device in the data storage 12b. That is, the relevant parameters of the motion are saved, e.g. the distance for running. Moreover, the control unit 10 is further configured to transmit the saved sensor data to at least a remote entity 80-1, 80-2 via the radio communication interface 30. The transmitting of the sensor data to a remote entity can take place momentarily as it is registered or at fixed intervals. The transmitting of the data may also be triggered by a movement or non-movement of the device. For example, the device can be configured to transmit the registered parameter values of the last power walk when the device has been in rest for 10 minutes. In another exemplary the transmitting takes place where the internet access is good.

These alternatives provides for an even more efficient utilization of the power resources of the motion detection device. Moreover, the user can according to some aspects decide how and when the results should be transmitted from the device. Furthermore, the user does not have to take any own action, in order for the motion detection device to send the information to a remote entity, this is performed autonomously. Hence, time consuming triggering of data transfer is reduced for the user and the data corresponding to his movement is directly available at the desired remote entity where e.g. a user-friendly interface is provided for.

In one embodiment at least one of the first movement pattern is associated with a movement pattern of a running laundry machine 5, cf. Figure 2. Hence, the motion detection can be designed to be robust and waterproof. As described above, in relation to the first movement pattern, the motion registration device will recognise the rotation of the laundry machine as a false alarm event and enter a motion registration device sleep mode for a sleep period corresponding to the time it takes for the laundry machine to finish. Thus, no motions are registered or measured by the sensors during this time and power is saved.

According to further aspects the present disclosure also relates to a piece of cloth 6 with a motion detecting device 1. The motion detecting device takes the form of the above described examples, embodiments and aspects of such device. The motion detection device can be attached to the garment in various way, permanently or removable.

Thereby, relevant movements of a user are directly registered by the autonomous motion detecting device when the piece of cloth is worn. Moreover, events that are not of interest for the user to register, i.e. false alarm events, are not registered and battery saving modes is entered by the device.

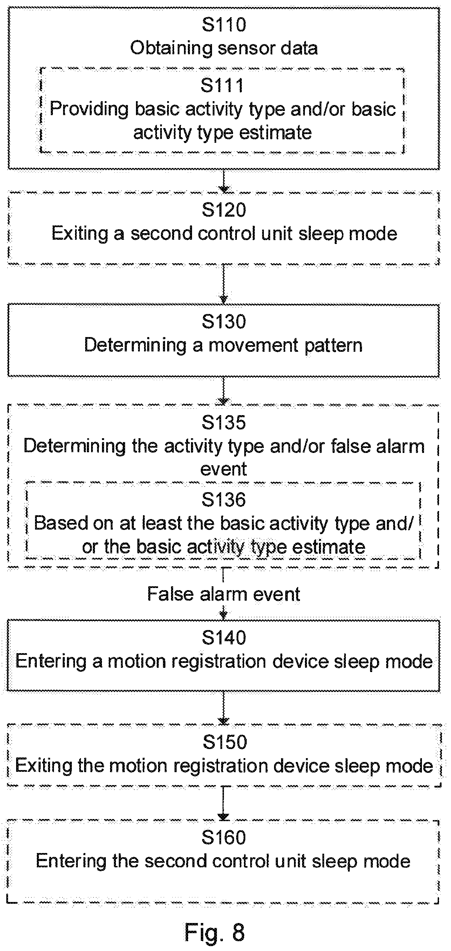

Figure 8 is a flowchart illustrating aspects of corresponding methods in a motion registration device.

The details of the method steps to be described are already discussed in relation to the corresponding devices above and will not be repeated. Moreover, one or more of the features, advantages and interrelationships discussed in relation to the different aspects, variants and embodiments of the motion registration device and its comprising parts are applicable to the corresponding aspects, variants, and embodiment of the method as described below.

The method comprises obtaining S110 sensor data, associated with a movement of the motion registration device, from one or more sensors. The method further comprises determining S130 a movement pattern of the motion registration device out of a plurality of movement patterns of the motion registration device based on at least the obtained sensor data. Moreover, at least a first movement pattern out of the plurality of movement patterns is associated with a false alarm event and at least a second movement pattern out of the plurality of movement patterns is associated with an activity type of the motion registration device. The method further comprises entering S140 a motion registration device sleep mode for a sleep period in response to determination of the at least first movement pattern.

Hence it is provided a layered method, combining external motion detection device with outer layers of refined motion detection.

According to further aspects, the plurality of movement patterns comprises a plurality of pre-defined movement patterns.

According to further aspects, the method further comprises determining S135 the activity type and/or the false alarm event based on at least the determined movement pattern.

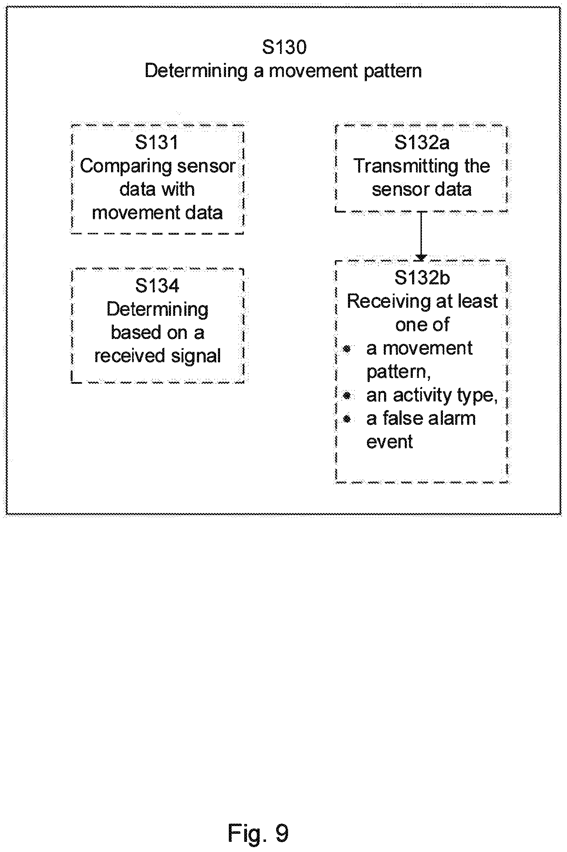

A flowchart illustrating aspects relating to the determining S130 movement pattern is shown in Figure 9.

According to further aspects, the determining S130 movement pattern of the motion registration device further comprises comparing S131 at least the obtained sensor data with movement data associated with the plurality of movement patterns.

According to further aspects, the determining S134 movement pattern of the motion registration device is further based on at least a received signal.

According to further aspects, the determining S130 movement pattern of the motion registration device further comprises transmitting S132a the obtained sensor data to at least a remote entity. The determining S130 movement pattern also comprises receiving S132b from the at least a remote entity at least one of a movement pattern out of the plurality of movement patterns, an activity type of the motion registration device and a false alarm event.

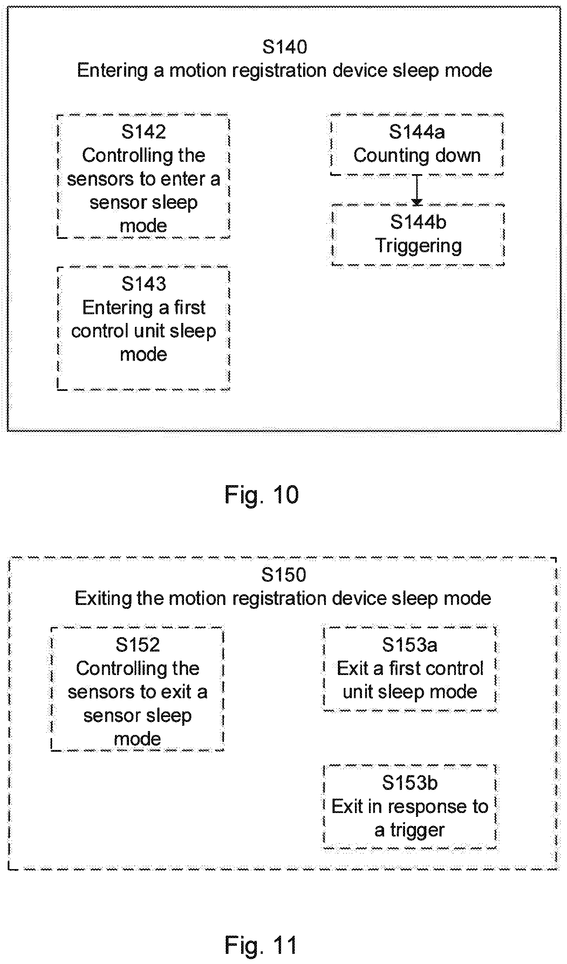

Flowcharts illustrating aspects relating to the entering S140 and exiting S150 a motion registration device sleep is shown in Figures 10 and 11.

According to further aspects, the method further comprises at least one of:
- controlling S142 the one or more sensors 20-1, 20-2, ..., 20-m to enter a sensor sleep mode when entering S140 the motion registration device sleep mode;
- entering S143 a first control unit sleep mode when entering S140 the motion registration device sleep mode;
- exiting S150 the motion registration device sleep mode;
- controlling S152 the one or more sensors 20-1, 20-2, ..., 20-m to exit the sensor sleep mode when exiting S150 the motion registration device sleep mode;
- exiting S153a the first control unit sleep mode when exiting S150 the motion registration device sleep mode; and
- exiting S153b the first control unit sleep mode in response to a trigger.

According to the previous aspects, the method may further comprise counting S144a down the sleep period and providing S144b the trigger to the control unit 10 to exit the control unit sleep mode when the sleep period has elapsed. Both the counting down and the providing the trigger are performed by a timer.

According to further aspects, the method further comprises exiting S120 a second control unit sleep mode in response to the obtaining S110 of sensor data and/or notification that sensor data is available from the one or more sensors. The method further comprises entering S160 the second control unit sleep mode in response to exiting the motion registration device sleep mode.

According to further aspects, each of the at least the first movement pattern is associated with a respective false alarm event, wherein the respective false alarm event is associated with a corresponding false alarm sleep period, and the sleep period is the corresponding false alarm sleep period.

According to further aspects, at least one of the first movement pattern is associated with a movement pattern of a running laundry machine.

The following exemplary relates to methods where sensor-HUBs are present. Hence, present is at least one of the one or more sensors which is configured to determine a basic activity type and a basic activity type estimate and transmit the determined basic activity type and the basic activity type estimate to the control unit. These optional steps are indicated as dashed boxes in Figure 8. Hence, according to further aspects, the method further comprises providing S111 at least a basic activity type and/or at least a basic activity type estimate from at least one of the one or more sensors. Moreover, the method further comprises determining S136 the activity type and/or false alarm event based on at least the basic activity type and/or the basic activity type estimate.

According to even further aspects, at least one of the one or more sensors is a Global Navigation Satellite System, GNSS, receiver.

According to the previous aspects, the sensor data may comprise received Global Navigation Satellite System, GNSS, signal strength.

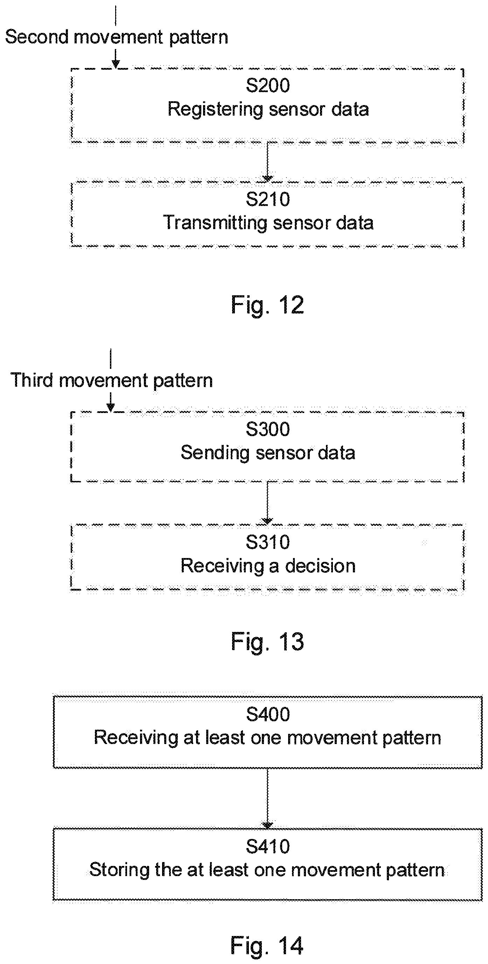

Figure 12 illustrates a flowchart relating to method steps occurring if the movement pattern is determined to be a second movement pattern.

According to further aspects, the method further comprises in response to determination of the second movement pattern registering S200 sensor data associated with a movement of the motion registration device.

According to the previous aspects, the method may further comprise transmitting S210 the registered sensor data to at least a remote entity.

Figure 13 illustrates a flowchart relating to method steps occurring if the movement pattern is determined to be a third movement pattern.

According to further aspects, at least a third movement pattern out of the plurality of movement patterns is associated with an undetermined movement pattern of the motion registration device. Moreover, in response to determination of the third movement pattern, the method further comprises sending S300 at least the obtained sensor data to at least a remote entity. It further comprises receiving S310 from the at least a remote entity a decision whether the movement of the motion registration device is of at least a first movement pattern or a second movement pattern.

Figure 14 illustrates a flowchart relating to method steps for updating the motion detection device.

According to further aspects, the method further comprises receiving S400 from the at least a remote entity at least one movement pattern and storing S410 the received at least one movement pattern.

According to some aspects, the present disclosure also relates to computer readable storage medium, having stored there on a computer program which, when executed in a motion registration device 1, causes the motion registration device 1 to execute the previously described methods.

The computer readable storage medium display advantages corresponding to the advantages already described in relation to the respective methods disclosed above.

The various aspects of motion detection devices and corresponding methods have above been described with reference to an exercising consumer. This usage should not been seen as limiting. Hence, the motion detection devices and methods described herein may also be utilized in other situations where sensor detectors are present.

Another exemplary utilization is to detect vibrations and impacts of e.g. machines and its movable parts, such as axes and bearings etc. According to some aspects, a vibration can be a false alarm event but it could also be an activity type of interest to log. Examples of vibrations that could be of interest to log are vibrations corresponding to damaged shafts or broken bearings, etc. One example is to log stationary vibrations of damaged shaft. For example, if a shaft is damaged there may be a need to log the corresponding recurrent vibration under a (longer) period of time (or until the shaft breaks) in order to characterize the vibration.

According to further aspects, referring to impact registration, it may be useful to register movements of freight containers. A false alarm event may in such situation correspond to loading / unloading (e.g. raising, lowering, moving vertically) of the container. A more dramatic event corresponding to e.g. a drop of the container or break in may correspond to a second movement pattern. According to some aspects, a detection of a second movement pattern may trigger an activity such as saving and/or transmitting an error message comprising the time and position of the event.

The description of the example embodiments provided herein have been presented for purposes of illustration. The description is not intended to be exhaustive or to limit example embodiments to the precise form disclosed, and modifications and variations are possible in light of the above teachings or may be acquired from practice of various alternatives to the provided embodiments. The examples discussed herein were chosen and described in order to explain the principles and the nature of various example embodiments and its practical application to enable one skilled in the art to utilize the example embodiments in various manners and with various modifications as are suited to the particular use contemplated. The features of the embodiments described herein may be combined in all possible combinations of methods, apparatus, modules, systems, and computer program products. It should be appreciated that the example embodiments presented herein may be practiced in any combination with each other.

It should be noted that the word "comprising" does not necessarily exclude the presence of other elements or steps than those listed and the words "a" or "an" preceding an element do not exclude the presence of a plurality of such elements. It should further be noted that any reference signs do not limit the scope of the claims, that the example embodiments may be implemented at least in part by means of both hardware and software, and that several "means", "units" or "devices" may be represented by the same item of hardware.

A "wireless device" as the term may be used herein, is to be broadly interpreted to include a radiotelephone having ability for Internet/intranet access, web browser, organizer, calendar, a camera (e.g., video and/or still image camera), a sound recorder (e.g., a microphone), and/or a Global Navigation Satellite System, GNSS, receiver; a personal communications system (PCS) user equipment that may combine a cellular radiotelephone with data processing; a personal digital assistant (PDA) that can include a radiotelephone or wireless communication system; a laptop; a camera (e.g., video and/or still image camera) having communication ability; and any other computation or communication device capable of transceiving, such as a personal computer, a home entertainment system, a television, etc. Furthermore, a device may be interpreted as any number of antennas or antenna elements.

Although the description is mainly given for a user equipment, as measuring or recording unit, it should be understood by the skilled in the art that "user equipment" is a non-limiting term which means any wireless device, terminal, or node capable of receiving in DL and transmitting in UL (e.g. PDA, laptop, mobile, sensor, fixed relay, mobile relay or even a radio base station, e.g. femto base station).

The various example embodiments described herein are described in the general context of method steps or processes, which may be implemented in one aspect by a computer program product, embodied in a computer-readable medium, including computer-executable instructions, such as program code, executed by computers in networked environments. A computer-readable medium may include removable and non-removable storage devices including, but not limited to, Read Only Memory (ROM), Random Access Memory (RAM), compact discs (CDs), digital versatile discs (DVD), etc. Generally, program modules may include routines, programs, objects, components, data structures, etc. that perform particular tasks or implement particular abstract data types. Computer-executable instructions, associated data structures, and program modules represent examples of program code for executing steps of the methods disclosed herein. The particular sequence of such executable instructions or associated data structures represents examples of corresponding acts for implementing the functions described in such steps or processes.

In the drawings and specification, there have been disclosed exemplary embodiments. However, many variations and modifications can be made to these embodiments. Accordingly, although specific terms are employed, they are used in a generic and descriptive sense only and not for purposes of limitation, the scope of the embodiments being defined by the following claims.

## Claims

1. A motion registration device (1), said device comprising:
a control unit (10);
one or more sensors (20-1, 20-2, ..., 20-m) configured to provide sensor data associated with a movement of the motion registration device (1) wherein at least one of the one or more sensors (20, 20-1, 20-2, ..., 20-m) further comprises a processor (21) and a sensor data storage (22), wherein the sensor data storage (22) is configured to store basic movement patterns, and wherein the processor (21) is configured to:
- determine a basic activity type and a basic activity type probability estimate comprising a probability of correctness based on the stored basic movement patterns;
- transmit the determined basic activity type and the basic activity type probability
estimate to the control unit (10);
wherein the control unit (10) is configured to:
- receive the sensor data sent from the one or more sensors (20-1, 20-2, ...., 20-m);
- receive at least the basic activity type and/or at least the basic activity type probability
estimate from at least one of the one or more sensors;
- determine a movement pattern of the motion registration device (1) out of a plurality of movement patterns of the motion registration device (1) based on at least the received sensor data, wherein at least a first movement pattern out of the plurality of movement patterns is associated with a false alarm event and at least a second movement pattern out of the plurality of movement patterns is associated with an activity type of the motion registration device so that the at least first or second movement pattern out of the plurality of movement patterns is determined;
- determine the activity type and/or false alarm event based on at least the basic activity type and/or the basic activity type probability estimate; and
- enter a motion registration device sleep mode and a control unit sleep mode for a sleep period in response to determination of the at least first movement pattern, wherein the control unit (10) is configured to control the one or more sensors (20-1, 20-2, ..., 20-m) to enter a sensor sleep mode when entering the motion registration device sleep mode wherein the one or more sensors (20-1, 20-2, ..., 20-m) are off; and wherein the control unit (10) is further configured to enter a control unit sleep mode when entering the motion registration device sleep mode wherein the control unit is turned off; and
wherein the motion registration device (1) further comprises a timer (13) configured to count down the sleep period and to provide a trigger to the control unit (10) to exit the control unit sleep mode and to exit the motion registration device sleep mode when the sleep period has elapsed.

2. A motion registration device (1) according to claim 1, wherein the plurality of movement patterns comprises a plurality of pre-defined movement patterns.

3. A motion registration device (1) according to any of claims 1-2, wherein the control unit (10) is further configured to:
- determine the activity type and/or the false alarm event based on at least the determined movement pattern.

4. A motion registration device (1) according to any of claims 1-3, wherein the control unit (10) comprises;
a data storage (12a) configured to store movement data associated with the plurality of movement patterns;
and wherein to determine a movement pattern further comprises:
- to compare at least the received sensor data with movement data stored in the data storage (12a).

5. A motion registration device (1) according to any of claims 1-4, further comprising;
a radio communication interface (30) configured to communicate with at least a remote entity (80-1, 80-2).

6. A motion registration device (1) according to claim 5, wherein to determine a movement pattern further comprises to:
- transmit from the sensors (20-1, 20-2, ..., 20-m) received sensor data to at least a remote entity (80-1, 80-2) via the radio communication interface (30), and
- receive from the at least a remote entity (80-1, 80-2) at least one of:
o a movement pattern out of the plurality of movement patterns;
o an activity type of the motion registration device; and
o a false alarm event.

7. A motion registration device (1) according to any of claims 1-6, wherein the at least first movement pattern is associated with a respective false alarm event, wherein the respective false alarm event is associated with a corresponding false alarm sleep period, and wherein the sleep period is the corresponding false alarm sleep period.

8. A piece of cloth (6) with a motion registration device (1), according to any of claims 1-7, attached to it.

9. A method in a motion registration device, the method comprising:
- obtaining (S110) sensor data, associated with a movement of the motion registration device, from one or more sensors;
- providing (Sill) at least a basic activity type and/or at least a basic activity type probability estimate comprising a probability of correctness from at least one of the one or more sensors;
- determining (S130) a movement pattern of the motion registration device out of a plurality of movement patterns of the motion registration device based on at least the obtained sensor data, wherein at least a first movement pattern out of the plurality of movement patterns is associated with a false alarm event and at least a second movement pattern out of the plurality of movement patterns is associated with an activity type of the motion registration device so that the at least first or second movement pattern out of the plurality of movement patterns is determined;
- determining (S136) the activity type and/or false alarm event based on at least the basic activity type and/or the basic activity type probability estimate;
- entering (S140) a motion registration device sleep mode and a control unit sleep mode for a sleep period in response to determination of the at least first movement pattern wherein the control unit (10) is configured to control the one or more sensors (20-1, 20-2, ..., 20-m) to enter a sensor sleep mode when entering the motion registration device sleep mode wherein the one or more sensors (20-1, 20-2, ..., 20-m) are off; and wherein the control unit (10) is further configured to enter a control unit sleep mode when entering the motion registration device sleep mode wherein the control unit is turned off; and
- exiting the control unit sleep mode and exiting the motion registration device sleep mode when the sleep period has elapsed.

10. A method according to claim 9, wherein the plurality of movement patterns comprise a plurality of pre-defined movement patterns.

11. A method according to any of claims 9-10, further comprising:
- determining (S135) the activity type and/or the false alarm event based on at least the determined movement pattern.

12. A method according to any of claims 9-11, wherein the determining (S130) movement pattern of the motion registration device further comprising:
- comparing (S131) at least the obtained sensor data with movement data associated with the plurality of movement patterns.

13. A method according to any of claims 9-12, wherein the determining (S130) movement pattern of the motion registration device further comprising:
- transmitting (S132a) the obtained sensor data to at least a remote entity, and
- receiving (S132b) from the at least a remote entity at least one of:
o a movement pattern out of the plurality of movement patterns;
o an activity type of the motion registration device; and
o a false alarm event.

14. A method according to any of the claims 9-13, wherein the at least first movement pattern is associated with a respective false alarm event, wherein the respective false alarm event is associated with a corresponding false alarm sleep period, and the sleep period is the corresponding false alarm sleep period.

15. A computer readable storage medium, having stored there on a computer program which, when executed in a motion registration device (1), causes the motion registration device (1) to execute the methods according to any of claims 9-14.

## Patentansprüche

1. Bewegungsregistrierungsvorrichtung (1), wobei die Vorrichtung Folgendes umfasst:
eine Steuereinheit (10);
einen oder mehrere Sensoren (20-1, 20-2, ..., 20-m), die konfiguriert sind, um Sensordaten bereitzustellen, die einer Bewegung der Bewegungsregistrierungsvorrichtung (1) zugeordnet sind, wobei zumindest einer von dem einen oder den mehreren Sensoren (20-1, 20-2, ..., 20-m) ferner einen Prozessor (21) und einen Sensordatenspeicher (22) umfasst, wobei der Sensordatenspeicher (22) konfiguriert ist, um Basisbewegungsmuster zu speichern, und wobei der Prozessor (21) für Folgendes konfiguriert ist:
- Bestimmen eines Basisaktivitätstyps und einer Basisaktivitätstypwahrscheinlichkeitsschätzung umfassend eine Wahrscheinlichkeit von Korrektheit basierend auf den gespeicherten Basisbewegungsmustern;
- Übertragen des bestimmten Basisaktivitätstyps und der bestimmten Basisaktivitätstypwahrscheinlichkeitsschätzung an die Steuereinheit (10);
wobei die Steuereinheit (10) für Folgendes konfiguriert ist:
- Empfangen der Sensordaten, die von dem einen oder den mehreren Sensoren (20-1, 20-2, ..., 20-m) gesendet werden;
- Empfangen von zumindest dem Basisaktivitätstyp und/oder zumindest der Basisaktivitätstypwahrscheinlichkeitsschätzung von zumindest einem von dem einen oder den mehreren Sensoren;
- Bestimmen eines Bewegungsmusters der Bewegungsregistrierungsvorrichtung (1) aus einer Vielzahl von Bewegungsmustern der Bewegungsregistrierungsvorrichtung (1) basierend auf zumindest den empfangenen Sensordaten, wobei zumindest ein erstes Bewegungsmuster aus der Vielzahl von Bewegungsmustern einem Falschalarmereignis zugeordnet ist und zumindest ein zweites Bewegungsmuster aus der Vielzahl von Bewegungsmustern einem Aktivitätstyp der Bewegungsregistrierungsvorrichtung zugeordnet ist, sodass das zumindest erste oder zweite Bewegungsmuster aus der Vielzahl von Bewegungsmustern bestimmt wird;
- Bestimmen des Aktivitätstyps und/oder Falschalarmereignisses basierend auf zumindest dem Basisaktivitätstyp und/oder der Basisaktivitätstypwahrscheinlichkeitsschätzung; und
- Eintreten in einen Ruhemodus der Bewegungsregistrierungsvorrichtung und einen Ruhemodus der Steuereinheit für einen Ruhezeitraum als Reaktion auf Bestimmung des zumindest ersten Bewegungsmusters, wobei die Steuereinheit (10) konfiguriert ist, um den einen oder die mehreren Sensoren (20-1, 20-2, ..., 20-m) zu steuern, um in einen Sensorruhemodus einzutreten, wenn in den Ruhemodus der Bewegungsregistrierungsvorrichtung eingetreten wird, wobei der eine oder die mehreren Sensoren (20-1, 20-2, ..., 20-m) aus sind; und wobei die Steuereinheit (10) ferner konfiguriert ist, um in einen Ruhemodus der Steuereinheit einzutreten, wenn in den Ruhemodus der Bewegungsregistrierungsvorrichtung eingetreten wird, wobei die Steuereinheit ausgeschaltet ist; und
wobei die Bewegungsregistrierungsvorrichtung (1) ferner einen Timer (13) umfasst, der konfiguriert ist, um den Ruhezeitraum hinunterzuzählen und um einen Auslöser an die Steuereinheit (10) bereitzustellen, um den Ruhemodus der Steuereinheit zu verlassen und um den Ruhemodus der Bewegungsregistrierungsvorrichtung zu verlassen, wenn der Ruhezeitraum verstrichen ist.

2. Bewegungsregistrierungsvorrichtung (1) nach Anspruch 1, wobei die Vielzahl von Bewegungsmustern eine Vielzahl von vordefinierten Bewegungsmustern umfasst.

3. Bewegungsregistrierungsvorrichtung (1) nach einem der Ansprüche 1 bis 2, wobei die Steuereinheit (10) ferner für Folgendes konfiguriert ist:
- Bestimmen des Aktivitätstyps und/oder des Falschalarmereignisses basierend auf zumindest dem bestimmten Bewegungsmuster.

4. Bewegungsregistrierungsvorrichtung (1) nach einem der Ansprüche 1 bis 3, wobei die Steuereinheit (10) Folgendes umfasst:
einen Datenspeicher (12a), der konfiguriert ist, um Bewegungsdaten zu speichern, die der Vielzahl von Bewegungsmustern zugeordnet sind;
und wobei das Bestimmen eines Bewegungsmusters ferner Folgendes umfasst:
- Vergleichen von zumindest den empfangenen Sensordaten mit Bewegungsdaten, die in dem Datenspeicher (12a) gespeichert sind.

5. Bewegungsregistrierungsvorrichtung (1) nach einem der Ansprüche 1 bis 4, ferner umfassend:
eine Funkkommunikationsschnittstelle (30), die konfiguriert ist, um mit zumindest einer entfernten Einheit (80-1, 80-2) zu kommunizieren.

6. Bewegungsregistrierungsvorrichtung (1) nach Anspruch 5, wobei das Bestimmen eines Bewegungsmusters ferner Folgendes umfasst:
- Übertragen, von den Sensoren (20-1, 20-2, ..., 20-m), von empfangenen Sensordaten an zumindest eine entfernte Einheit (80-1, 80-2) über die Funkkommunikationsschnittstelle (30), und
- Empfangen, von der zumindest einen entfernten Einheit (80-1, 80-2), von zumindest einem von:
o einem Bewegungsmuster aus der Vielzahl von Bewegungsmustern;
o einem Aktivitätstyp der Bewegungsregistrierungsvorrichtung; und
o einem Falschalarmereignis.

7. Bewegungsregistrierungsvorrichtung (1) nach einem der Ansprüche 1 bis 6, wobei das zumindest eine erste Bewegungsmuster einem jeweiligen Falschalarmereignis zugeordnet ist, wobei das jeweilige Falschalarmereignis einem entsprechenden Falschalarmruhezeitraum zugeordnet ist, und wobei der Ruhezeitraum der entsprechende Falschalarmruhezeitraum ist.

8. Kleidungsstück (6) mit einer Bewegungsregistrierungsvorrichtung (1) nach einem der Ansprüche 1 bis 7, die daran angebracht ist.

9. Verfahren in einer Bewegungsregistrierungsvorrichtung, wobei das Verfahren Folgendes umfasst:
- Erhalten (S110) von Sensordaten, die einer Bewegung der Bewegungsregistrierungsvorrichtung zugeordnet sind, von einem oder mehreren Sensoren;
- Bereitstellen (S111) von zumindest einem Basisaktivitätstyp und/oder zumindest einer Basisaktivitätstypwahrscheinlichkeitsschätzung umfassend eine Wahrscheinlichkeit von Korrektheit von zumindest einem von dem einen oder den mehreren Sensoren;
- Bestimmen (S130) eines Bewegungsmusters der Bewegungsregistrierungsvorrichtung aus einer Vielzahl von Bewegungsmustern der Bewegungsregistrierungsvorrichtung basierend auf zumindest den erhaltenen Sensordaten, wobei zumindest ein erstes Bewegungsmuster aus der Vielzahl von Bewegungsmustern einem Falschalarmereignis zugeordnet ist und zumindest ein zweites Bewegungsmuster aus der Vielzahl von Bewegungsmustern einem Aktivitätstyp der Bewegungsregistrierungsvorrichtung zugeordnet ist, sodass das zumindest erste oder zweite Bewegungsmuster aus der Vielzahl von Bewegungsmustern bestimmt wird;
- Bestimmen (S136) des Aktivitätstyps und/oder Falschalarmereignisses basierend auf zumindest dem Basisaktivitätstyp und/oder der Basisaktivitätstypwahrscheinlichkeitsschätzung;
- Eintreten (S140) in einen Ruhemodus der Bewegungsregistrierungsvorrichtung und einen Ruhemodus der Steuereinheit für einen Ruhezeitraum als Reaktion auf Bestimmung des zumindest ersten Bewegungsmusters, wobei die Steuereinheit (10) konfiguriert ist, um den einen oder die mehreren Sensoren (20-1, 20-2, ..., 20-m) zu steuern, um in einen Sensorruhemodus einzutreten, wenn in den Ruhemodus der Bewegungsregistrierungsvorrichtung eingetreten wird, wobei der eine oder die mehreren Sensoren (20-1, 20-2, ..., 20-m) aus sind; und wobei die Steuereinheit (10) ferner konfiguriert ist, um in einen Ruhemodus der Steuereinheit einzutreten, wenn in den Ruhemodus der Bewegungsregistrierungsvorrichtung eingetreten wird, wobei die Steuereinheit ausgeschaltet ist; und
- Verlassen des Ruhemodus der Steuereinheit und Verlassen des Ruhemodus der Bewegungsregistrierungsvorrichtung, wenn der Ruhezeitraum verstrichen ist.

10. Verfahren nach Anspruch 9, wobei die Vielzahl von Bewegungsmustern eine Vielzahl von vordefinierten Bewegungsmustern umfasst.

11. Verfahren nach einem der Ansprüche 9 bis 10, ferner umfassend:
- Bestimmen (S135) des Aktivitätstyps und/oder des Falschalarmereignisses basierend auf zumindest dem bestimmten Bewegungsmuster.

12. Verfahren nach einem der Ansprüche 9 bis 11, wobei das Bestimmen (S130) des Bewegungsmusters der Bewegungsregistrierungsvorrichtung ferner Folgendes umfasst:
- Vergleichen (S131) von zumindest den erhaltenen Sensordaten mit Bewegungsdaten, die der Vielzahl von Bewegungsmustern zugeordnet sind.

13. Verfahren nach einem der Ansprüche 9 bis 12, wobei das Bestimmen (S130) des Bewegungsmusters der Bewegungsregistrierungsvorrichtung ferner Folgendes umfasst:
- Übertragen (S132a) der erhaltenen Sensordaten an zumindest eine entfernte Einheit, und
- Empfangen (S132b), von der zumindest einen entfernten Einheit, von zumindest einem von:
o einem Bewegungsmuster aus der Vielzahl von Bewegungsmustern;
o einem Aktivitätstyp der Bewegungsregistrierungsvorrichtung; und
o einem Falschalarmereignis.

14. Verfahren nach einem der Ansprüche 9 bis 13, wobei das zumindest eine erste Bewegungsmuster einem jeweiligen Falschalarmereignis zugeordnet ist, wobei das jeweilige Falschalarmereignis einem entsprechenden Falschalarmruhezeitraum zugeordnet ist, und der Ruhezeitraum der entsprechende Falschalarmruhezeitraum ist.

15. Computerlesbares Speichermedium, auf dem ein Computerprogramm gespeichert ist, das, wenn es in einer Bewegungsregistrierungsvorrichtung (1) ausgeführt wird, die Bewegungsregistrierungsvorrichtung (1) dazu veranlasst, die Verfahren nach einem der Ansprüche 9 bis 14 auszuführen.

## Revendications

1. Dispositif d'enregistrement de mouvement (1), ledit dispositif comprenant :
une unité de commande (10) ;
un ou plusieurs capteurs (20-1, 20-2,..., 20-m) configurés pour fournir des données de capteur associées à un mouvement du dispositif d'enregistrement de mouvement (1) dans lequel au moins un des un ou plusieurs capteurs (20, 20-1, 20-2,..., 20-m) comprend en outre un processeur (21) et une mémoire de données de capteur (22), dans lequel la mémoire de données de capteur (22) est configurée pour stocker des modèles de mouvement de base, et dans lequel le processeur (21) est configuré pour :
- déterminer un type d'activité de base et une estimation de probabilité de type d'activité de base comprenant une probabilité d'exactitude sur la base des modèles de mouvement de base stockés ;
- transmettre le type d'activité de base déterminé et l'estimation de probabilité de type d'activité de base à l'unité de commande (10) ;
dans lequel l'unité de commande (10) est configurée pour :
- recevoir les données de capteur envoyées par les un ou plusieurs capteurs (20-1, 20-2,...., 20-m) ;
- recevoir au moins le type d'activité de base et/ou au moins l'estimation de probabilité de type d'activité de base d'au moins un parmi les un ou plusieurs capteurs ;
- déterminer un modèle de mouvement du dispositif d'enregistrement de mouvement (1) parmi une pluralité de modèles de mouvement du dispositif d'enregistrement de mouvement (1) sur la base d'au moins les données de capteur reçues, dans lequel au moins un premier modèle de mouvement parmi la pluralité de modèles de mouvement est associé à un événement de fausse alarme et au moins un second modèle de mouvement parmi la pluralité de modèles de mouvement est associé à un type d'activité du dispositif d'enregistrement de mouvement de sorte que l'au moins un premier ou second modèle de mouvement parmi la pluralité de modèles de mouvement est déterminé ;
- déterminer le type d'activité et/ou l'événement de fausse alarme sur la base d'au moins le type d'activité de base et/ou l'estimation de probabilité de type d'activité de base ; et
- entrer dans un mode de veille de dispositif d'enregistrement de mouvement et dans un mode de veille d'unité de commande pendant une période de veille en réponse à la détermination de l'au moins un premier modèle de mouvement, dans lequel l'unité de commande (10) est configurée pour commander les un ou plusieurs capteurs (20-1, 20-2,..., 20-m) pour entrer dans un mode de veille de capteur lors de l'entrée dans le mode de veille de dispositif d'enregistrement de mouvement dans lequel les un ou plusieurs capteurs (20-1, 20-2,..., 20-m) sont désactivé ;
et dans lequel l'unité de commande (10) est en outre configurée pour entrer dans un mode de veille d'unité de commande lors de l'entrée dans le mode de veille de dispositif d'enregistrement de mouvement dans lequel l'unité de commande est éteinte ; et
dans lequel le dispositif d'enregistrement de mouvement (1) comprend en outre une minuterie (13) configurée pour décompter la période de veille et pour fournir un déclencheur à l'unité de commande (10) pour quitter le mode de veille d'unité de commande et pour quitter le mode de veille de dispositif d'enregistrement de mouvement lorsque la période de veille est écoulée.

2. Dispositif d'enregistrement de mouvement (1) selon la revendication 1, dans lequel la pluralité de modèles de mouvement comprend une pluralité de modèles de mouvement prédéfinis.

3. Dispositif d'enregistrement de mouvement (1) selon l'une quelconque des revendications 1 à 2, dans lequel l'unité de commande (10) est en outre configurée pour :
- déterminer le type d'activité et/ou l'événement de fausse alarme sur la base d'au moins le modèle de mouvement déterminé.

4. Dispositif d'enregistrement de mouvement (1) selon l'une quelconque des revendications 1 à 3, dans lequel l'unité de commande (10) comprend ;
une mémoire de données (12a) configurée pour stocker des données de mouvement associées à la pluralité de modèles de mouvement ; et dans lequel pour déterminer un modèle de mouvement comprend en outre :
- pour comparer au moins les données de capteur reçues avec des données de mouvement stockées dans la mémoire de données (12a) .

5. Dispositif d'enregistrement de mouvement (1) selon l'une quelconque des revendications 1 à 4, comprenant en outre ;
une interface de communication radio (30) configurée pour communiquer avec au moins une entité distante (80-1, 80-2).

6. Dispositif d'enregistrement de mouvement (1) selon la revendication 5, dans lequel la détermination d'un modèle de mouvement comprend en outre de :
- transmettre depuis les capteurs (20-1, 20-2,..., 20-m) les données de capteur reçues à au moins une entité distante (80-1, 80-2) via l'interface de communication radio (30), et
- recevoir de l'au moins une entité distante (80-1, 80-2) au moins l'un parmi :
o un modèle de mouvement parmi la pluralité de modèles de mouvement ;
o un type d'activité du dispositif d'enregistrement de mouvement ; et
∘ un événement de fausse alarme.

7. Dispositif d'enregistrement de mouvement (1) selon l'une quelconque des revendications 1 à 6, dans lequel l'au moins un premier modèle de mouvement est associé à un événement de fausse alarme respectif, dans lequel l'événement de fausse alarme respectif est associé à une période de veille de fausse alarme correspondante, et dans lequel la période de veille est la période de veille de fausse alarme correspondante.

8. Morceau de tissu (6) avec un dispositif d'enregistrement de mouvement (1), selon l'une quelconque des revendications 1 à 7, attaché à celui-ci.

9. Procédé dans un dispositif d'enregistrement de mouvement, le procédé comprenant :
- l'obtention (S110) de données de capteur, associées à un mouvement du dispositif d'enregistrement de mouvement, à partir d'un ou plusieurs capteurs ;
- la fourniture (S111) d'au moins un type d'activité de base et/ou d'au moins une estimation de probabilité de type d'activité de base comprenant une probabilité d'exactitude à partir d'au moins l'un des un ou plusieurs capteurs ;
- la détermination (S130) d'un modèle de mouvement du dispositif d'enregistrement de mouvement parmi une pluralité de modèles de mouvement du dispositif d'enregistrement de mouvement sur la base d'au moins les données de capteur obtenues, dans lequel au moins un premier modèle de mouvement parmi la pluralité de modèles de mouvement est associé à un événement de fausse alarme et au moins un second modèle de mouvement parmi la pluralité de modèles de mouvement est associé à un type d'activité du dispositif d'enregistrement de mouvement de sorte que l'au moins un premier ou second modèle de mouvement parmi la pluralité de modèles de mouvement est déterminé ;
- la détermination (S136) du type d'activité et/ou de l'événement de fausse alarme sur la base d'au moins le type d'activité de base et/ou l'estimation de probabilité de type d'activité de base ;
- l'entrée (S140) d'un mode de veille de dispositif d'enregistrement de mouvement et d'un mode de veille d'unité de commande pendant une période de veille en réponse à la détermination de l'au moins un premier modèle de mouvement, dans lequel l'unité de commande (10) est configurée pour commander les un ou plusieurs capteurs (20-1, 20-2,..., 20-m) pour entrer dans un mode de veille de capteur lors de l'entrée dans le mode de veille de dispositif d'enregistrement de mouvement dans lequel les un ou plusieurs capteurs (20-1, 20-2,..., 20-m) sont éteints ; et dans lequel l'unité de commande (10) est en outre configurée pour entrer dans un mode de veille d'unité de commande lors de l'entrée dans le mode de veille de dispositif d'enregistrement de mouvement dans lequel l'unité de commande est éteinte ; et
- la sortie du mode de veille d'unité de commande et la sortie du mode de veille de dispositif d'enregistrement de mouvement lorsque la période de veille s'est écoulée.

10. Procédé selon la revendication 9, dans lequel la pluralité de modèles de mouvement comprend une pluralité de modèles de mouvement prédéfinis.

11. Procédé selon l'une quelconque des revendications 9 à 10, comprenant en outre :
- la détermination (S135) du type d'activité et/ou de l'événement de fausse alarme sur la base d'au moins le modèle de mouvement déterminé.

12. Procédé selon l'une quelconque des revendications 9 à 11, dans lequel la détermination (S130) du modèle de mouvement du dispositif d'enregistrement de mouvement comprend en outre :
- la comparaison (S131) d'au moins les données de capteur obtenues avec des données de mouvement associées à la pluralité de modèles de mouvement.

13. Procédé selon l'une quelconque des revendications 9 à 12, dans lequel la détermination (S130) du modèle de mouvement du dispositif d'enregistrement de mouvement comprend en outre :
- la transmission (S132a) des données de capteur obtenues à au moins une entité distante, et
- la réception (S132b) de l'au moins une entité distante d'au moins l'un parmi :
∘ un modèle de mouvement parmi la pluralité de modèles de mouvement ;
∘ un type d'activité du dispositif d'enregistrement de mouvement ; et
∘ un événement de fausse alarme.

14. Procédé selon l'une quelconque des revendications 9 à 13, dans lequel l'au moins un premier modèle de mouvement est associé à un événement de fausse alarme respectif, dans lequel l'événement de fausse alarme respectif est associé à une période de veille de fausse alarme correspondante, et la période de veille est la période de veille de fausse alarme correspondante.

15. Support de stockage lisible par ordinateur, stocké sur un programme informatique qui, lorsqu'il est exécuté dans un dispositif d'enregistrement de mouvement (1), amène le dispositif d'enregistrement de mouvement (1) à exécuter les procédés selon l'une quelconque des revendications 9 à 14.
